# EUROPÄISCHE PATENTSCHRIFT

(11) **EP 2 906 196 B1**
(45) Veröffentlichungstag und Bekanntmachung des Hinweises auf die Patenterteilung: **31.08.2022**
(21) Anmeldenummer: 13759745.6
(22) Anmeldetag: 10.09.2013
(51) Int. Cl.: A61K 9/00, A61K 9/06, A61K 47/36, A61K 47/32, A61K 47/14, A61K 47/12, A61K 47/10, A61K 31/121, A61K 31/075, A61K 31/045, A61K 31/085, A61K 31/11, A61K 31/12, A61K 31/166, A61K 31/215, A61K 31/22, A61K 31/4402, A61K 45/06, A61P 17/00

(54) **KOSMETISCHE ODER DERMATOLOGISCHE ZUBEREITUNG ZUR PROPHYLAXE UND/ODER BEHANDLUNG ATOPISCHER DERMATITIS**
COSMETIC OR DERMATOLOGICAL PREPARATION FOR PROPHYLAXIS AND/OR TREATMENT OF ATOPIC DERMATITIS
PRÉPARATION COSMÉTIQUE OU DERMATOLOGIQUE POUR LA PRÉVENTION ET/OU LE TRAITEMENT DE LA DERMATITE ATOPIQUE

(30) Priorität: 15.10.2012 DE 102012218733
(43) Veröffentlichungstag der Anmeldung: 19.08.2015
(73) Patentinhaber: Beiersdorf AG, 20253 Hamburg (DE)
(72) Erfinder: NEUFANG, Gitta, Hamburg 20149 (DE); SCHLÄGER, Torsten, 22297 Hamburg (DE); VOIGT, Nadine, 22111 Hamburg (DE); WORTHMANN, Anne-Christin, 25474 Hasloh (DE)
(74) Vertreter: Beiersdorf AG
(86) Internationale Anmeldenummer: PCT/EP2013/068674
(87) Internationale Veröffentlichungsnummer: WO 2014/060150

(56) Entgegenhaltungen:
- WO-A1-98/01134
- WO-A1-2006/134013
- WO-A1-2009/127282
- DE-A1- 19 824 681
- DE-U1-202007 015 195
- US-A1- 2008 253 973
- SONALI S. BHARATE ET AL: "Modulation of Thermoreceptor TRPM8 by Cooling Compounds", ACS CHEMICAL NEUROSCIENCE, Bd. 3, Nr. 4, 18. April 2012 (2012-04-18), Seiten 248-267, XP055090754, ISSN: 1948-7193, DOI: 10.1021/cn300006u

## Beschreibung

Die vorliegende Erfindung betrifft eine Zubereitung zur äußerlichen Anwendung, welche als ein Therapeutikum, zur prophylaktischen Behandlung oder zur Verbesserung atopischer Dermatitis verwendet wird.

Eine atopische Dermatitis ist bekannt als eine Typ-I-allergische Reaktion reagierend auf IgE. Verschiedene äußerliche Zubereitungen zur therapeutischen Behandlung atopischer Hauterkrankung wurden bis jetzt entwickelt, welche eine Verbindung mit einer Aktivität zur Inhibierung von PCA-Reaktionen, welche auf IgE reagiert, als einen aktiven Inhaltsstoff umfasst.

In der praktischen Anwendung ist jedoch keine bekannt, die adäquat wirksam ist, während Steroide, wie beispielsweise adrenocorticoidale Hormone, weiterhin die Masse äußerlicher Zubereitungen für eine therapeutische Behandlung atopischer Hauterkrankungen ausmachen.

Obwohl die Steroide, die gegenwärtig für atopische Dermatitis verwendet werden, welche für Hautkrankheiten und andere Hautkrankheiten angewendet werden, exzellente therapeutische Wirksamkeit aufweisen, wenn sie über eine lange Zeitperiode verwendet wird, werden systemische Nebenwirkungen, umfassend die funktionale Suppression des Hypothalamus, der Hypophyse und des Adrenocortex produziert.

Trotz der Tatsache, dass sie als äußerliche Zubereitungen verwendet werden, rufen sie zusätzlich häufig lokale Nebenwirkungen in der Form von Hautsymptomen, wie beispielsweise Verschlimmerung von Hautinfektionen und Akne-Charakteristika adrenocorticaler Hormone hervor. Es wurde ebenfalls im Verlauf der Verabreichung auf Narben, Leberflecke und Sommersprossen sowie auf Probleme in Bezug auf die Reaktion nach Beendigung der Verabreichung hingewiesen.

Aufgrund der obigen Probleme wurden Immunsuppressiva, Antihistaminika und Antiallergika usw. als therapeutische Agenzien für atopische Dermatitis entwickelt. Immunsuppressiva involvieren jedoch Probleme, wie z. B. Verschlimmerung bakterieller. Hautinfektionen, und Antihistaminika involvieren Probleme nachteiliger Nebenwirkungen, wie z. B. Medikamentenausschlag.

In Bezug auf atopische Dermatitis, deren Ursache bis jetzt noch nicht identifiziert wurde, leiden Patienten mit Symptomen sowie ihre Familienmitglieder unter Juckreiz. Hautjucken oder Pruritus ist ein verbreitetes dermatologisches Symptom, das Ursache für erhebliche Beeinträchtigungen sowohl beim Menschen als auch bei Tieren sein kann. Pruritus ist oft mit entzündlichen Hauterkrankungen assoziiert, die durch Überempfindlichkeitsreaktionen.

Bestehende Behandlungen, die für die Behandlung von Pruritus verwendet wurden, beinhalten die Verwendung von Corticosteroiden und Antihistaminen. Jedoch ist bei diesen beiden Behandlung das Auftreten unerwünschter Nebenwirkungen bekannt. Andere Therapien, die angewendet wurden, beinhalten die Verwendung von Nahrungsergänzungen mit essentiellen Fettsäuren, obwohl diese mit den Nachteilen verbunden sind, langsam zu wirken und nur begrenzte Wirksamkeit gegenüber allergischer Dermatitis zu bieten. Eine Vielzahl von Aufweichmitteln, wie etwa streichfähiges Paraffin, Glycerin und Lanolin, werden ebenfalls verwendet, jedoch mit begrenztem Erfolg.

Es besteht somit ein fortbestehender Bedarf an alternativen und/oder verbesserten Behandlungen für Hautjucken (Pruritus).

Da definitive Behandlungsmethoden bis jetzt noch nicht von Gesundheitsversorgeinstituten, beispielsweise Universitäten, Krankenhäuser usw. etabliert wurden, gibt es einen dringenden Bedarf für die Entwicklung einer effektiven äußerlichen Zubereitung für eine therapeutische oder prophylaktische Behandlung atopischer Dermatitis, die möglichst frei von Nebenwirkungen ist, um die Steroid-Typ-antiinflammatorischen äußerlichen Zubereitungen zu ersetzen

Die TRP-Kanäle (englisch *transient receptor potential channels*) sind eine umfangreiche Familie von zellulären lonenkanälen, die in sieben Unterfamilien gegliedert werden kann. Die Homologie (DNA- bzw. Aminosäuresequenz-Verwandtschaft) zwischen den Unterfamilien ist nur mäßig ausgeprägt. Gemeinsam ist allen Mitgliedern, dass sie 6 Transmembranregionen besitzen und durchlässig für Kationen sind.

Man unterscheidet folgende Unterfamilien:
klassische Unterfamilie (TRPC)
Vanilloid-Rezeptor-Unterfamilie (TRPV)
Melastatin Unterfamilie (TRPM)
NOMPC-Unterfamilie (TRPN)
ANKTM1-Unterfamilie (TRPA)
Mucolipin-Unterfamilie (TRPML)
Polycystin-Unterfamilie (TRPP)

Man vermutet, dass jeweils vier Protein-Untereinheiten in der Zellmembran einen lonenkanal mit einer zentralen Pore bilden (Tetramer). Sowohl Homotetramere (vier gleiche Untereinheiten) als auch Heterotetramere (Tetramere aus mehreren verschiedenen Untereinheiten) sind möglich.

TRP-Kanäle sind entwicklungsgeschichtlich sehr alt (sie finden sich z. B. bereits in Hefezellen). Die Funktion der meisten TRP-Kanäle ist allerdings noch weitgehend ungeklärt. Insekten beispielsweise benötigen TRP-Kanäle zum Sehen und bei der Schmerzwahrnehmung.

Beim Menschen spielen TRP-Kanäle eine wichtige Rolle bei der Wahrnehmung von Geschmack (süß, sauer, umami), Pheromonen, Temperatur (warm, heiß, kalt), Schmerz u. a.

Der TRPM-8 Rezeptor beispielsweise wird auch als "cold and menthol receptor 1", auch CMR1, also Kalt-und-Menthol-Rezeptor genannt. Agonisten für den TRPM-8 Rezeptor, beispielsweise Linalool, Geraniol, Hydroxycitronellal, WS-3 (N-Ethyl-p-menthan-3-carboxamid, WS-23 (2-Isopropyl-N,2,3-trimethylbutyramide), Frescolat MAG (1,4-Dioxaspiro[4.5]decan-2-methanol), Frescolat ML (Menthyllactat), Coolact P (5-Methyl-2-prop-1-en-2-ylcyclohexan-1-ol) und Cooling Agent 10 (Menthoxypropanediol)rufen ein Kältegefühl hervor

Es war indessen erstaunlich und für den Fachmann nicht vorhersehbar, dass kosmetische Zubereitungen, enthaltend Wirkstoffkombinationen aus Licochalcon A, Coolant XL ((1R,2S,5R)-N-(2-(2-pyridinyl)ethyl)-2-ispropyl-5-methylcyclohexancarboxamid) und Cooling Agent 10 (Menthoxypropandiol) und 1,2-Decandiol geeignet sind und die Probleme des Standes der Technik lösen würden.

Cooling Agent 10 (Menthoxypropandiol) hat die Struktur 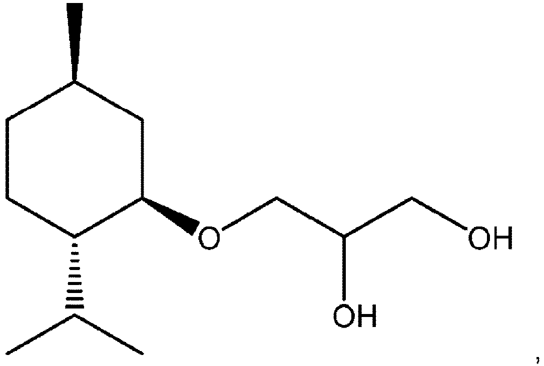 Erfindungsgemäß wird das Cooling Agent 10 (Menthoxypropandiol) in kosmetischen Zusammensetzungen eingesetzt einem Gehalt von 0,005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-%, ganz besonders vorteilhaft 0,3 - 3,0 Gew.-%, jeweils bezogen auf das Gesamtgewicht der Zusammensetzung.

Es ist im Sinne der Erfindung von großem Vorteil, wenn den Zubereitungen gemäß der Erfindung antimikrobiell wirksame (insbesondere antibakteriell wirksame) Substanzen und/oder antierythematös wirksame bzw. entzündungshemmende Substanzen beigefügt werden.

Als antimikrobiell wirksamen Substanz oder Substanzen kann oder können im Sinne der vorliegenden Erfindung beispielsweise vorteilhaft gewählt werden eine oder mehrere Substanzen aus der Gruppe der Polyole

Erfindungsgemäß können die Polyole können vorteilhaft gewählt werden aus der Gruppe der mindestens bifunktionellen Alkohole. Vorteilhaft sind insbesondere die Polyole, gewählt aus der folgenden Gruppe:
Ethylenglycol, Polyethylenglycole mit Molmassen bis zu ca. 2.000, Propylenglycol-1,2, Polypropylenglycole-1,2 mit Molmassen bis zu ca. 2.000, Propylenglycol-1,3, Polypropylenglycole-1,3 mit Molmassen bis zu ca. 2.000, Butylenglycol-1,2, Polybutylenglycole1,2 mit Molmassen bis zu ca. 2.000, Butylenglycol-1,3, Polybutylenglycole-1,3 mit Molmassen bis zu ca. 2.000, Butylenglycol-1,4, Polybutylenglycole-1,4 mit Molmassen bis zu ca. 2.000, Butylenglycol-2,3, Polybutylenglycole-2,3 mit Molmassen bis zu ca. 2.000, α,ω-Alkandiole, Glycerin, Diglycerin, Triglycerin, Tetraglycerin und Pentaglycerin, wobei die Oligoglycerine, sowie deren Fettsäureester aus über eine oder mehrere Etherbrücken kondensierten Glycerineinheiten zusammengesetzt sind, beispielsweise wie folgt:

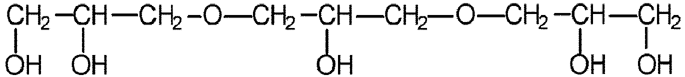

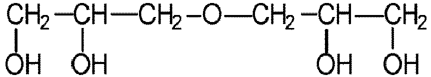

Ganz bevorzugte Polyole werden gewählt aus der Gruppe der vicinalen Diole, also der 1,2-Alkandiole. Unter diesen wiederum sind bevorzugt 1,2-Pentandiol, 1,2-Hexandiol. 1,2-Heptandiol, 1,2-Octandiol. 1,2-Nonandiol und 1,2-Decandiol.

Erfindungsgemäß wird 1,2-Decandiol bevorzugt in kosmetischen Zusammensetzungen eingesetzt einem Gehalt von 0,005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an 1,2-Decandiol ganz besonders vorteilhaft 0,3 - 3,0 Gew.-%, jeweils bezogen auf das Gesamtgewicht der Zusammensetzung.

Vorteilhafte antierythematös oder anti-entzündliche Wirkstoffe im Sinne der vorliegenden Erfindung sind beispielsweise Licochalcon A, Lignan, Chroman- oder Isoflavonoide, pentacyclische Triterpene (z.B. Ursolsäure, Glycyrrhetinsäure), Hamamelis Kamillenextrakte oder Bisabolol, Allantoin, Calendula Extrakte und/oder Panthenol.

Licochalcon A, zeichnet sich durch die folgende Strukturformel aus:

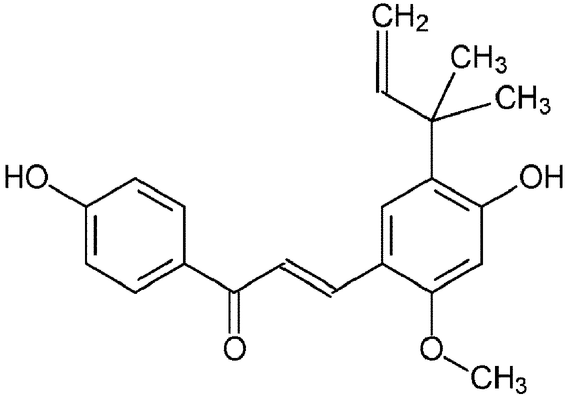

Es wird in Form wäßriger Extrakten aus der Wurzel der Pflanzenart *Glycyrrhiza inflata* gewonnen, die wie das in Europa offizinelle Süßholz *Glycyrrhiza glabra* der Gattung *Glycyrrhiza* angehört, die wiederum zur Pflanzenfamilie der *Fabaceae* (Erbsengewächse) gehört. Die Droge *Radix Glycyrrhizae inflatae,* d.h., die Wurzel der Pflanze, ist, beispielsweise in der fernöstlichen Medizin, gebräuchlich. Die Verwendung der Droge als Entzündungshemmer ist ebenfalls bekannt.

Erfindungsgemäß wird Licochalcon A in kosmetischen oder dermatologischen Zusammensetzungen eingesetzt einem Gehalt von 0,005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an einem oder mehreren erfindungsgemäß verwendeten antientzündlichen Wirkstoffen, ganz besonders vorteilhaft 0,3 - 3,0 Gew.-%, jeweils bezogen auf das Gesamtgewicht der Zusammensetzung.

Vorteilhaft kann der erfindungsgemäß verwendete Wirkstoff eingearbeitet werden in übliche kosmetische und dermatologische Zubereitungen, welche in verschiedenen Formen vorliegen können. So können sie z. B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-ÖI-in-Wasser (W/O/W) oder Öl-in-Wasser-in-Öl (O/W/O), eine Hydrodispersion oder Lipodispersion, eine Pickering-Emulsion, ein Gel, einen festen Stift oder auch ein Aerosol darstellen.

Erfindungsgemäße Emulsionen im Sinne der vorliegenden Erfindung, z. B. in Form einer Creme, einer Lotion, einer kosmetischen Milch sind vorteilhaft und enthalten z. B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmann natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt.

Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leicht flüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wässrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

### Wirknachweis:

Das Ziel der vorliegenden Studie war die Untersuchung der Wirksamkeit einer optimierten Hautpflegeformel bestehend aus Licochalcone A (Glycyrrhiza Inflata Wurzelextrakt), Decandiol, Cooling Agent 1 (Coolant XL) und Cooling Agent 10 (Coolact CA10) als antiinflammatorische, anti-bakterielle und anti-pruritogene Substanzen, an Patienten mit atopischer Dermatitis mit schwacher bis moderater Ausprägung.

Unter Verwendung eines randomisierten, verblindeteten (Untersuchender), kontrollierten, zweiarmigen Studiendesigns, war es das Ziel, das Cortison-Einspar-Potential und die Nicht-Unterlegenheit des Prüfmusters bei einer zweimal täglich über vier Wochen andauernden Anwendung im Vergleich zu einer einwöchigen Behandlung mit 1% Hydrocortison (HC), gefolgt von einer Anwendung des Prüfmusters für weitere drei Wochen, zu bewerten. Die Studienergebnisse basierten auf einer vom Dermatologen kontrollierten Beurteilung des Schweregrads der klinischen Zeichen der atopischen Dermatitis, der Kolonisierung der Hautläsionen mit *S.aureus,* biotechnologischer Messung von Parametern der epidermalen Barrierefunktion sowie der Juckreizintensität im Testareal.

Die Ergebnisse der Studie liefern Hinweise dafür, dass die im Studienprotokoll definierten Ziele erreicht wurden. Ähnlich wie 1% Hydrocortison zeigte die alleinige Applikation des Prüfmusters eine signifikante Reduktion des lokalen SCORADs, der Erytheme, des läsionalen Haut-TEWLs (Transepidermaler Wasserverlust) sowie eine signifikante Reduktion der Kolonisierung der Hautläsionen mit *S.aureus.* Während die Anwendung von 1% HC innerhalb der ersten Studienwoche stärkere Veränderungen der oben genannten Parameter induzierte, außer TEWL an Tag 7 für läsionale Haut, gab es zu keinem Zeitpunkt signifikante Unterschiede zwischen den untersuchten Armen.

Besonders hervorzuheben ist, dass die Reduktionen des lokalen SCORADs, des läsionalen Haut-TEWLs, der Erytheme und der *S*.*aureus*-Besiedlung, bestimmt als prozentuale Veränderung im Vergleich zum Startwert am entsprechenden Arm A und Arm B, am Ende der Studie vergleichbar waren. Daraus kann geschlussfolgert werden, dass eine Nicht-Unterlegenheit der alleinigen Anwendung des Prüfmusters im Vergleich zu einem Behandlungsansatz mit 1% Hydrocortison und dem Prüfmuster vorliegt. Dieses Resultat zeigt einen potentiellen Cortison einsparenden Effekt des Prüfmusters, das der Menge des benutzten topischen Steroids pro Patient in der ersten Studienwoche entspricht (6,08g 1% HC/ 7 Tage).

Die Anwendung des Prüfmusters oder 1% HC in der ersten Studienwoche resultierte in Unterschieden bezüglich der ausgeübten Effekte auf die Hautbefeuchtung. Während die Anwendung von 1% HC die läsionale Haut nur geringfügig befeuchtete, erzielte die Anwendung des Prüfmusters 37175-10 einen signifikanten Anstieg der Hautfeuchte am jeweiligen Test-Arm. Wichtig zu beachten ist, dass der Anstieg der läsionalen Hautfeuchte am Ende der Studie für beide Test-Arme A und B signifikant war, wodurch der Schluß nahe liegt, dass der beobachtete Zugewinn an Hautfeuchte nach der diskontinuierlichen Anwendung von 1% HC am jeweiligen Test-Arm der Anwendung des Prüfmusters 37175-10 zuzuschreiben ist. Das Prüfmuster war im Allgemeinen gut hautverträglich. Die am häufigsten benannte subjektive Einschätzung nach Anwendung der Prüfmuster war ein Brennen, das von 16,7 % der Probanden unabhängig vom Test-Arm und Zeitpunkt verspürt wurde. Hautrötung, Krustenbildung, Hauttrockenheit, Hautspannen oder andere subjektive Einschätzungen wurden nur in vereinzelten Fällen genannt.

Die Studienergebnisse zeigen demnach, dass die zweimal tägliche Anwendung des Prüfmusters 37175-10 durch eine Reduktion des klinischen Schweregrads (lokaler SCORAD), eine Verbesserung der Hautbarriere-Parameter, eine Reduktion der pathogenen Baktierien-Besiedlung sowie einem messbaren Cortison-Einspar-Potential einen wesentlichen Nutzen für die Behandlung akut-ekzematöser Läsionen von Patienten mit schwach bis moderat ausgeprägter atopischer Dermatitis darstellt.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

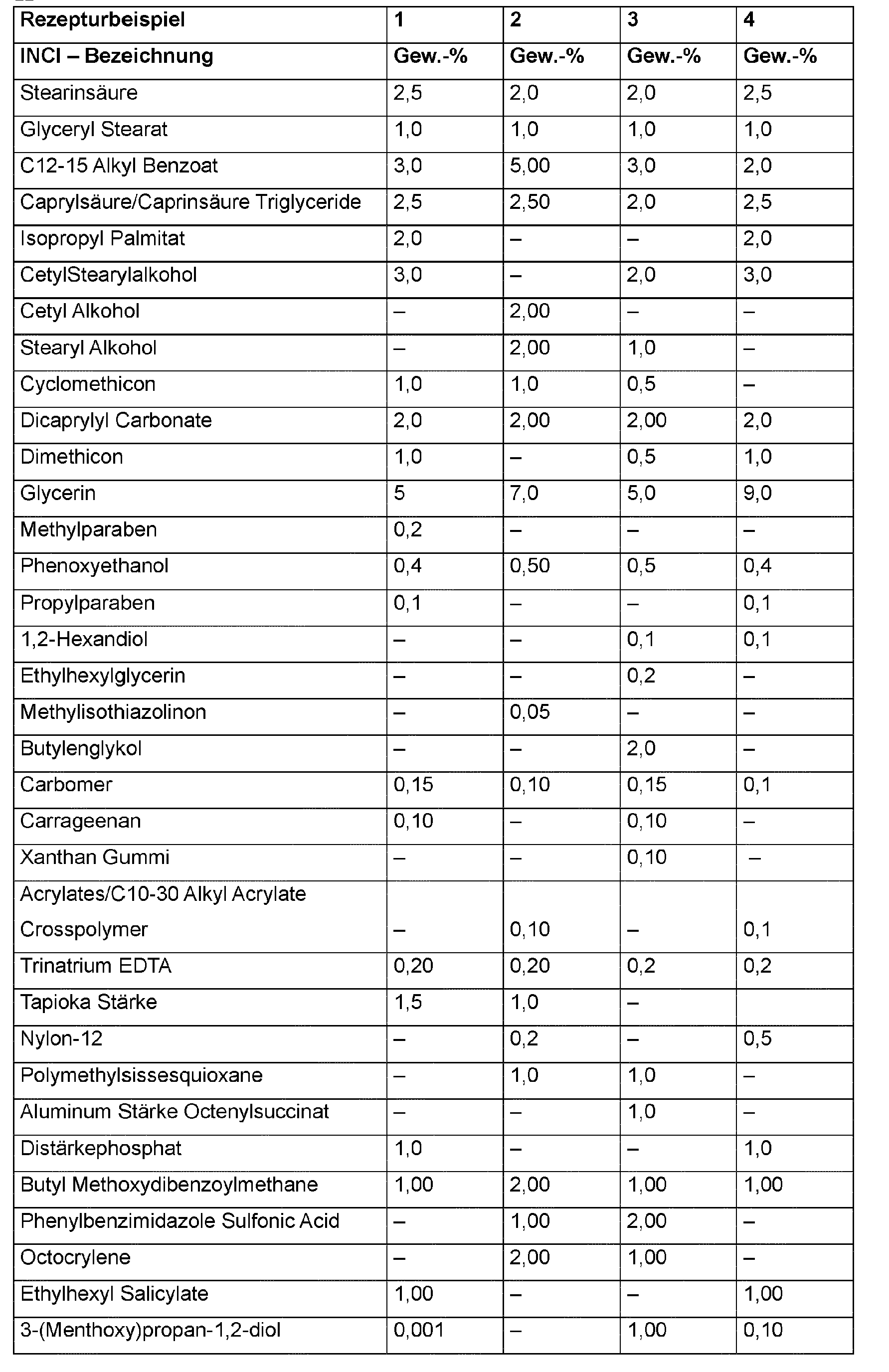

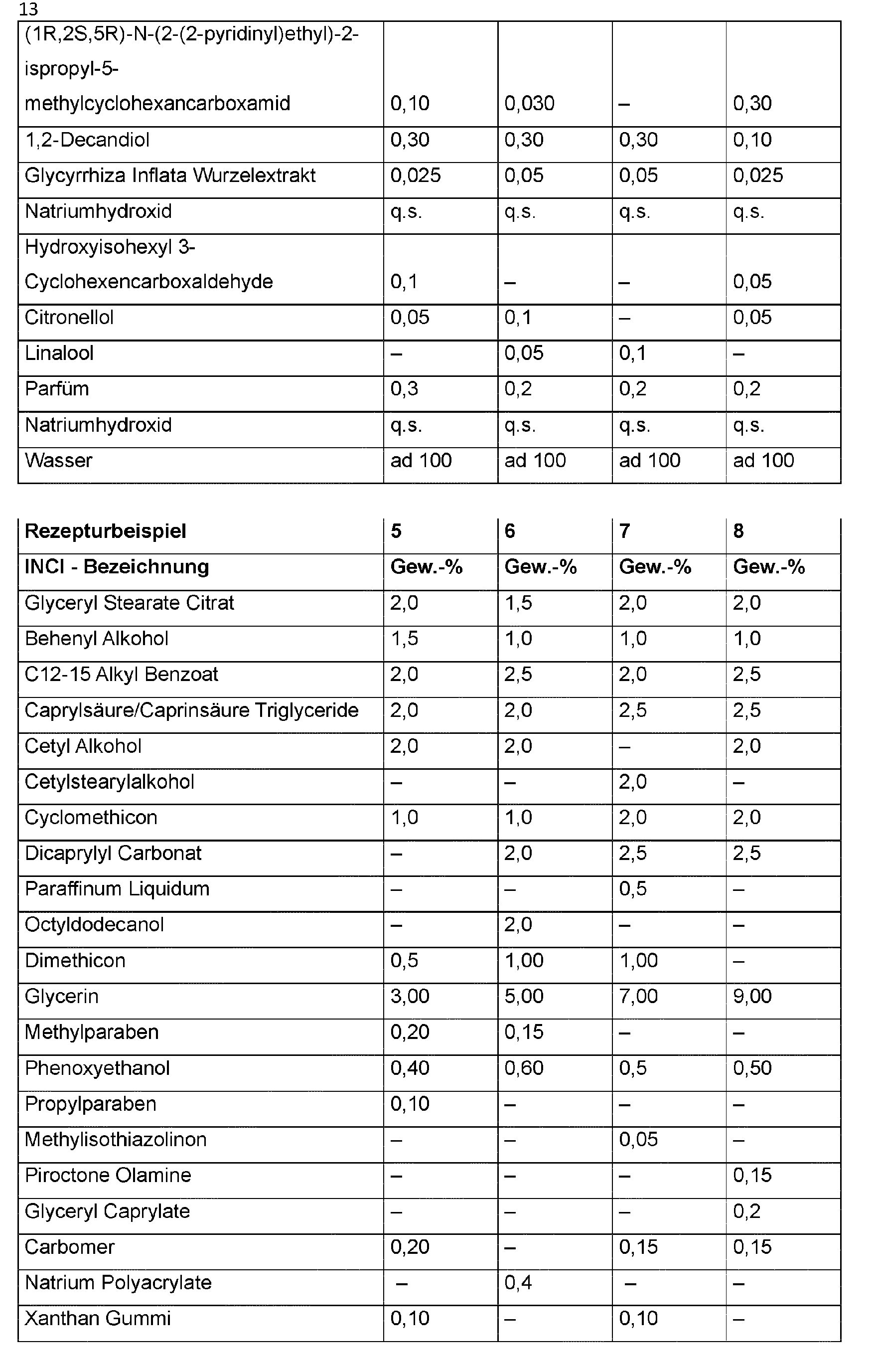

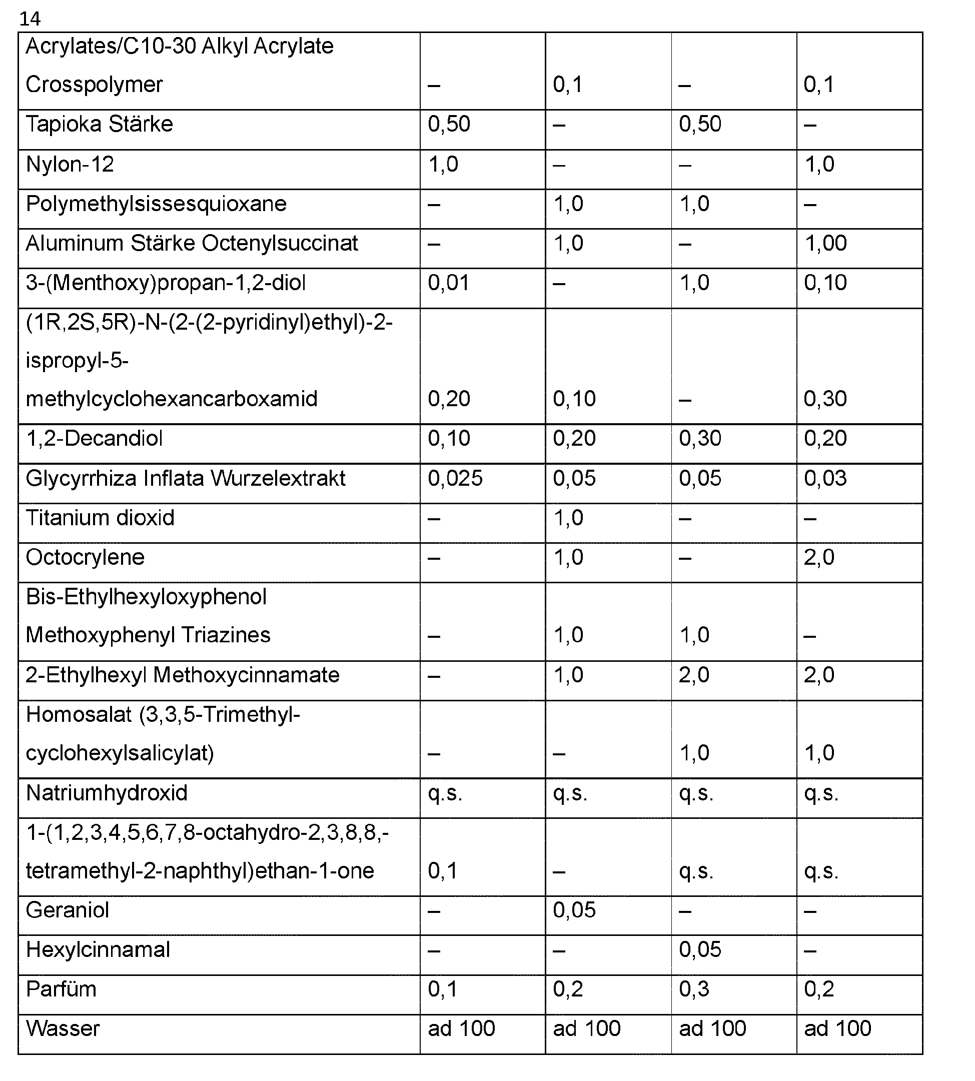

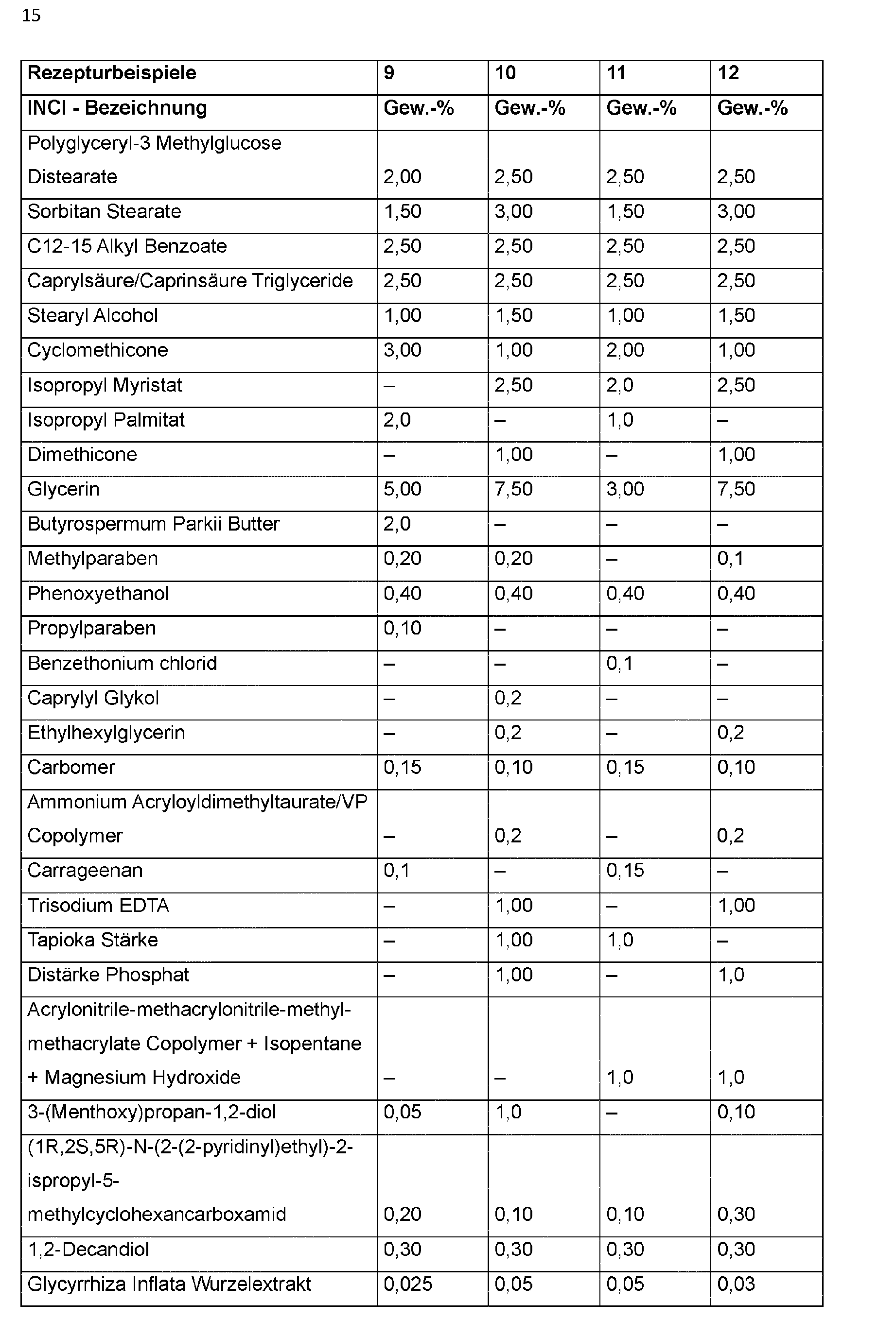

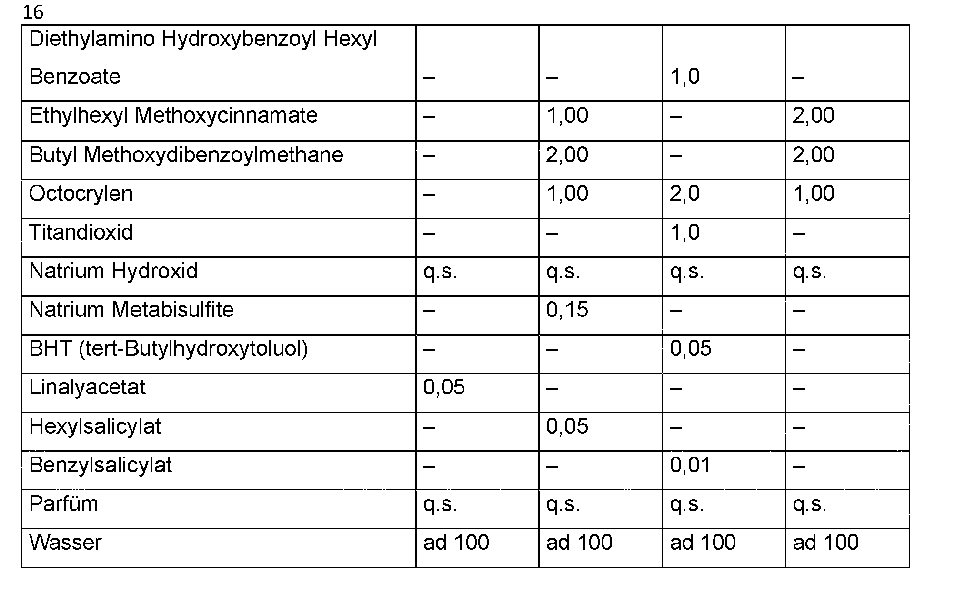

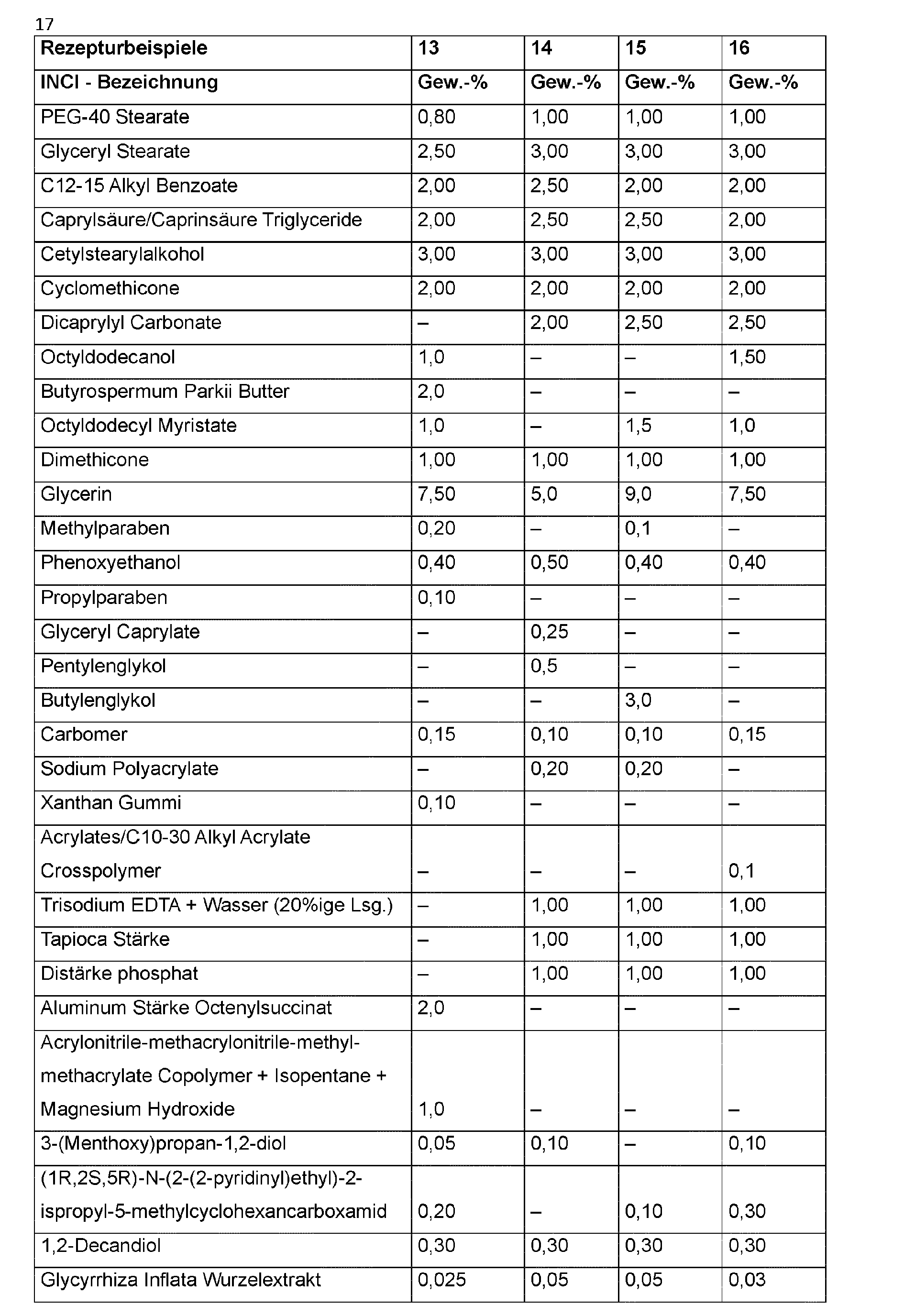

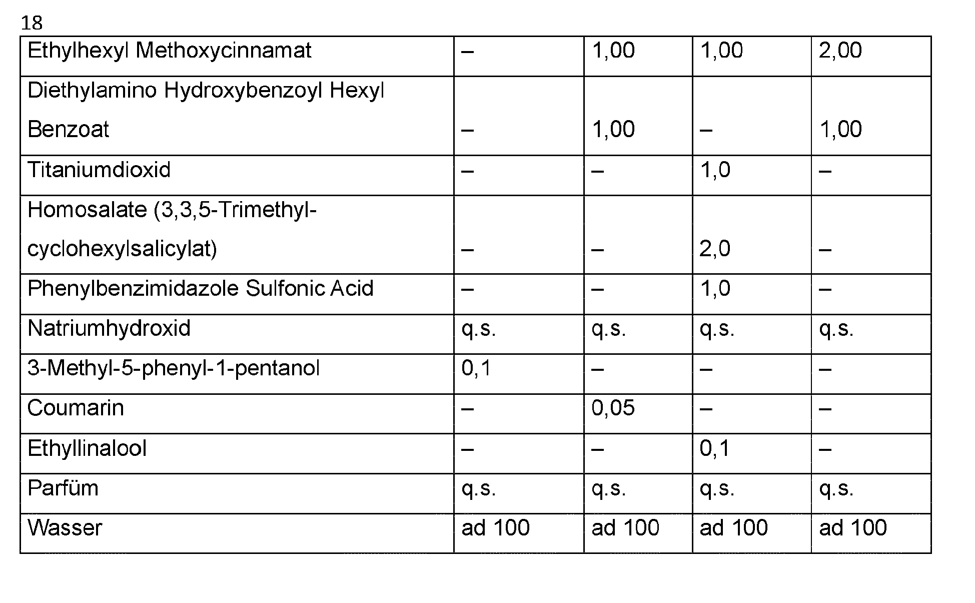

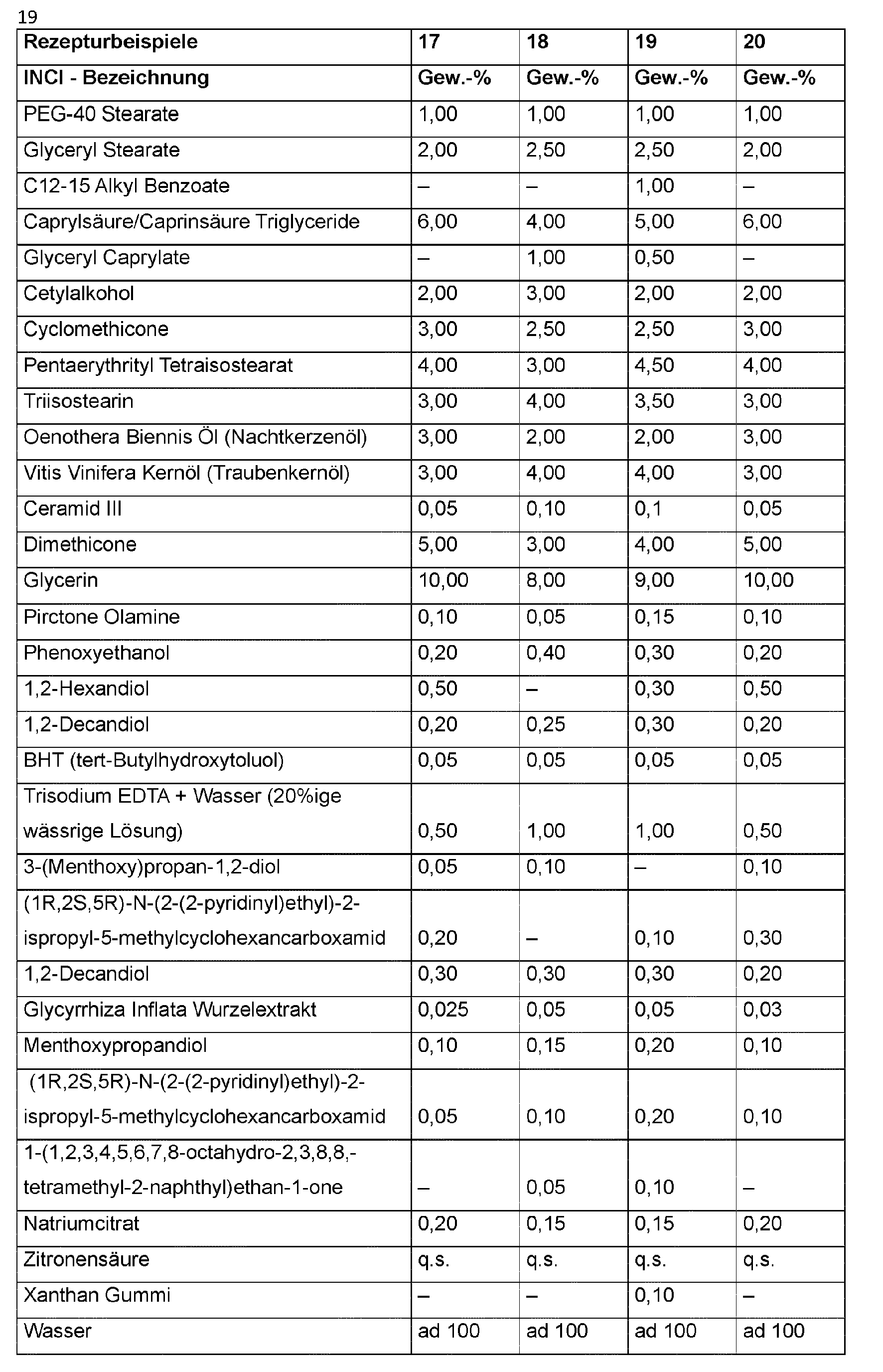

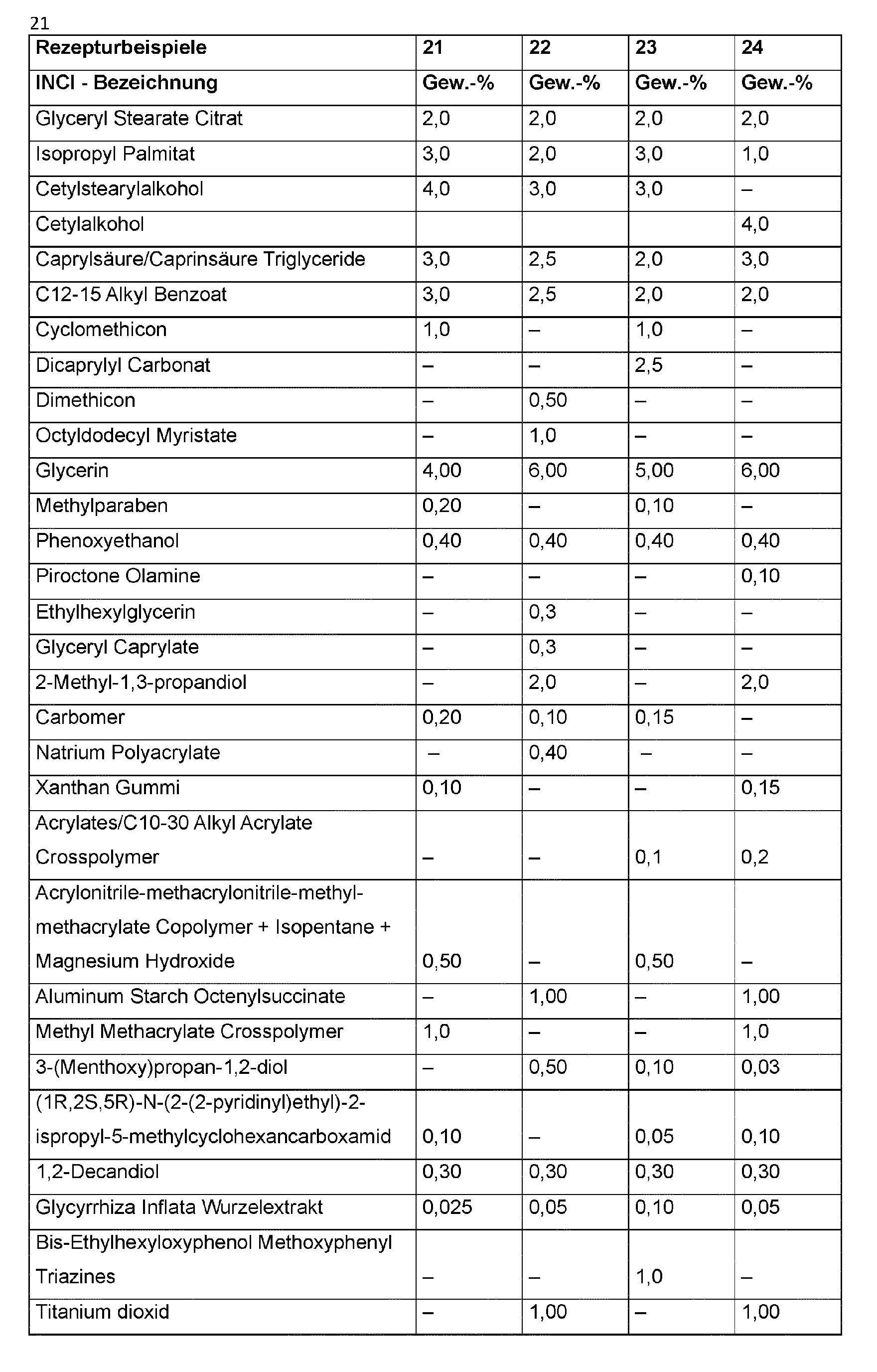

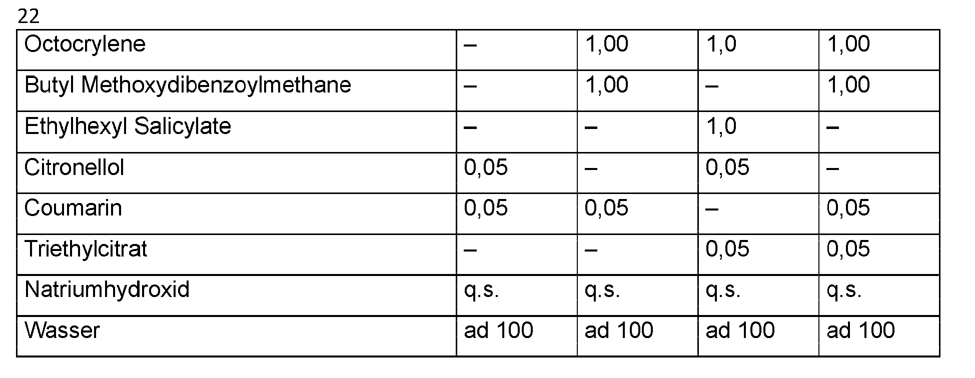

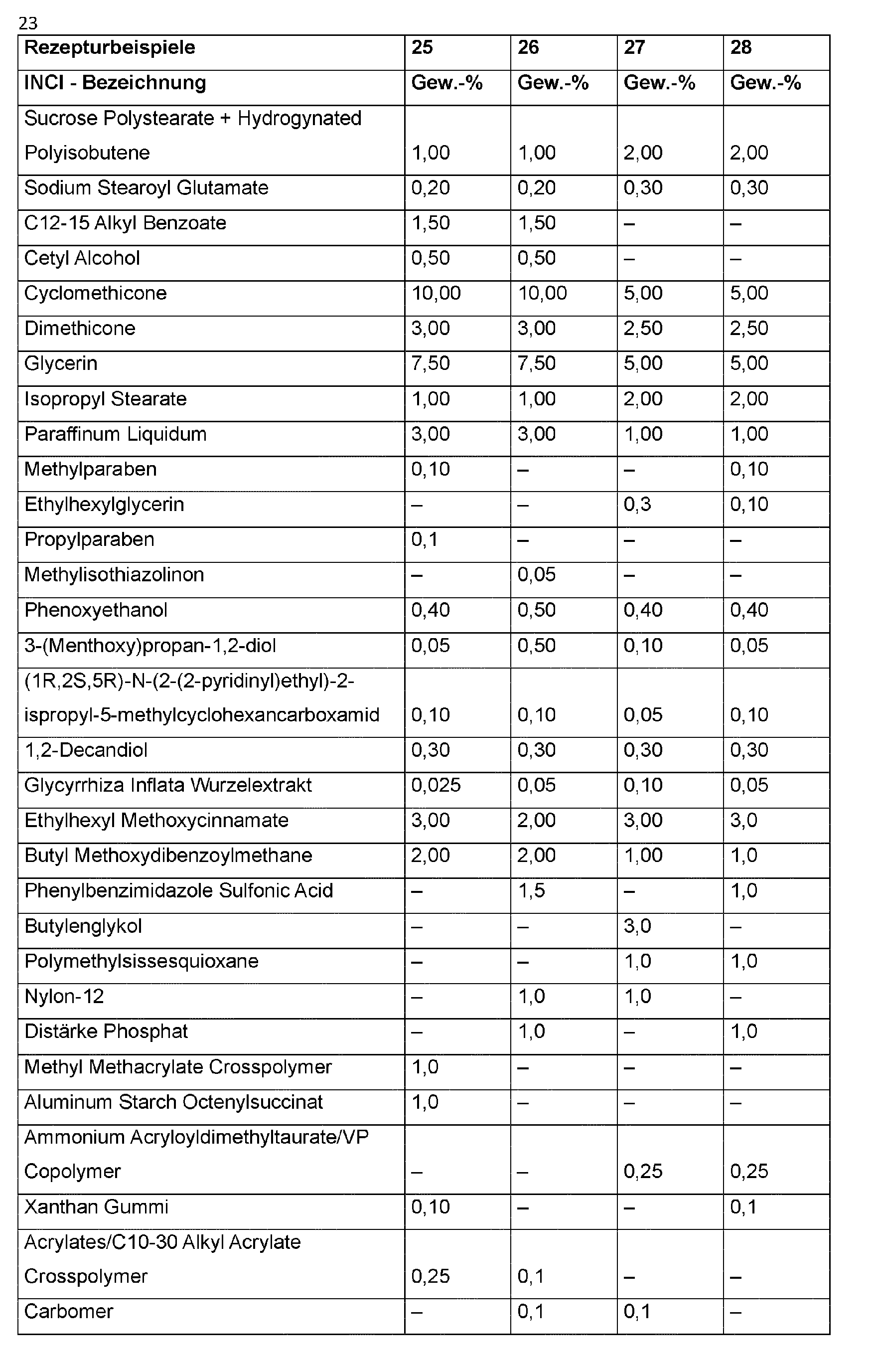

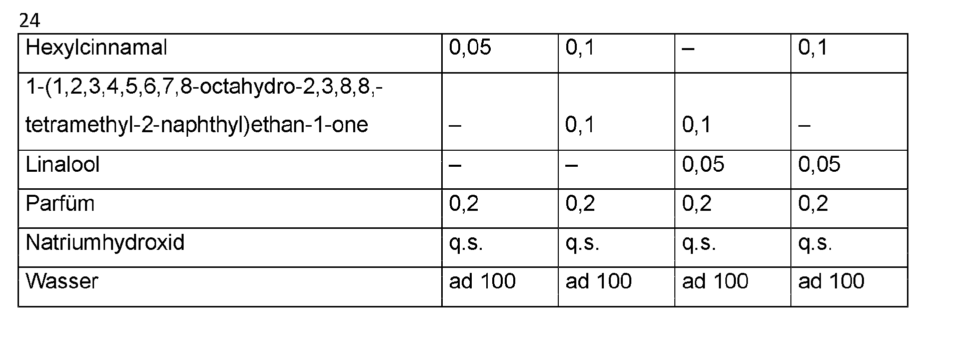

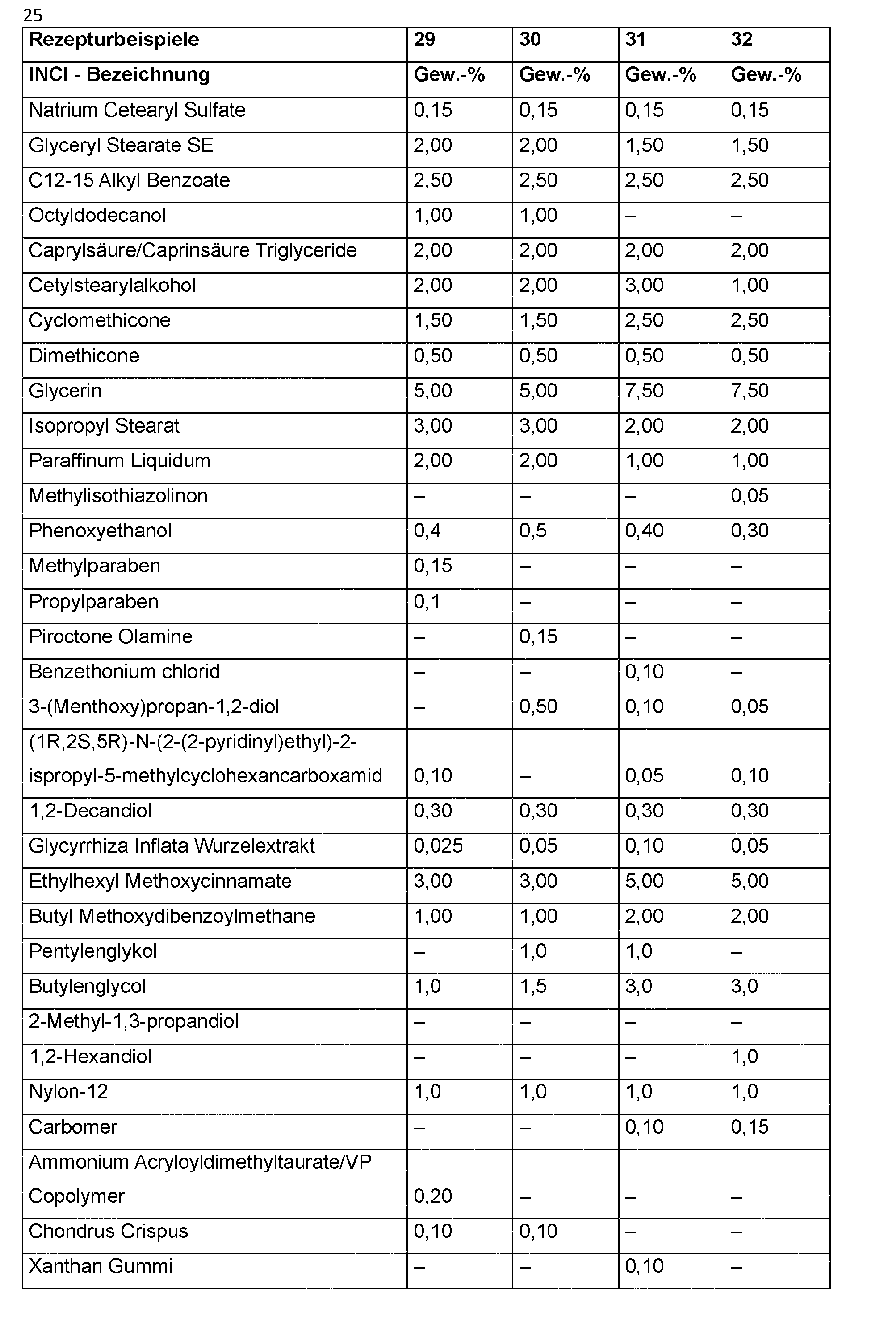

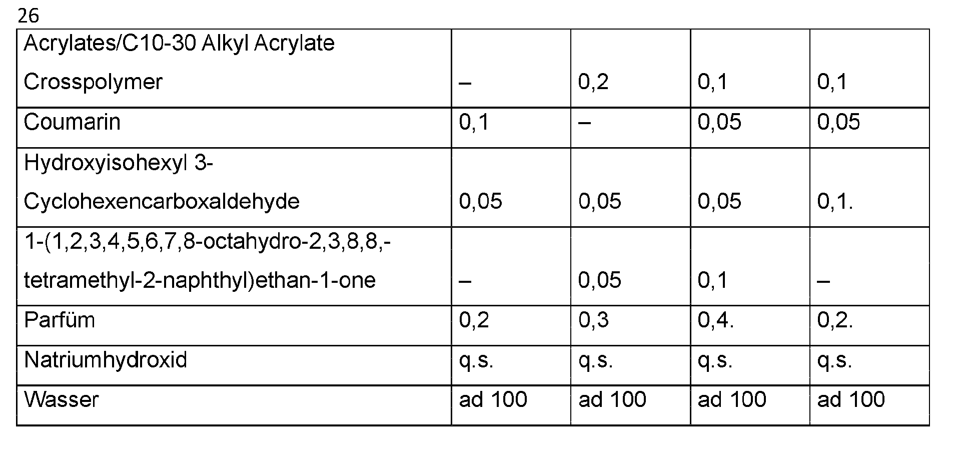

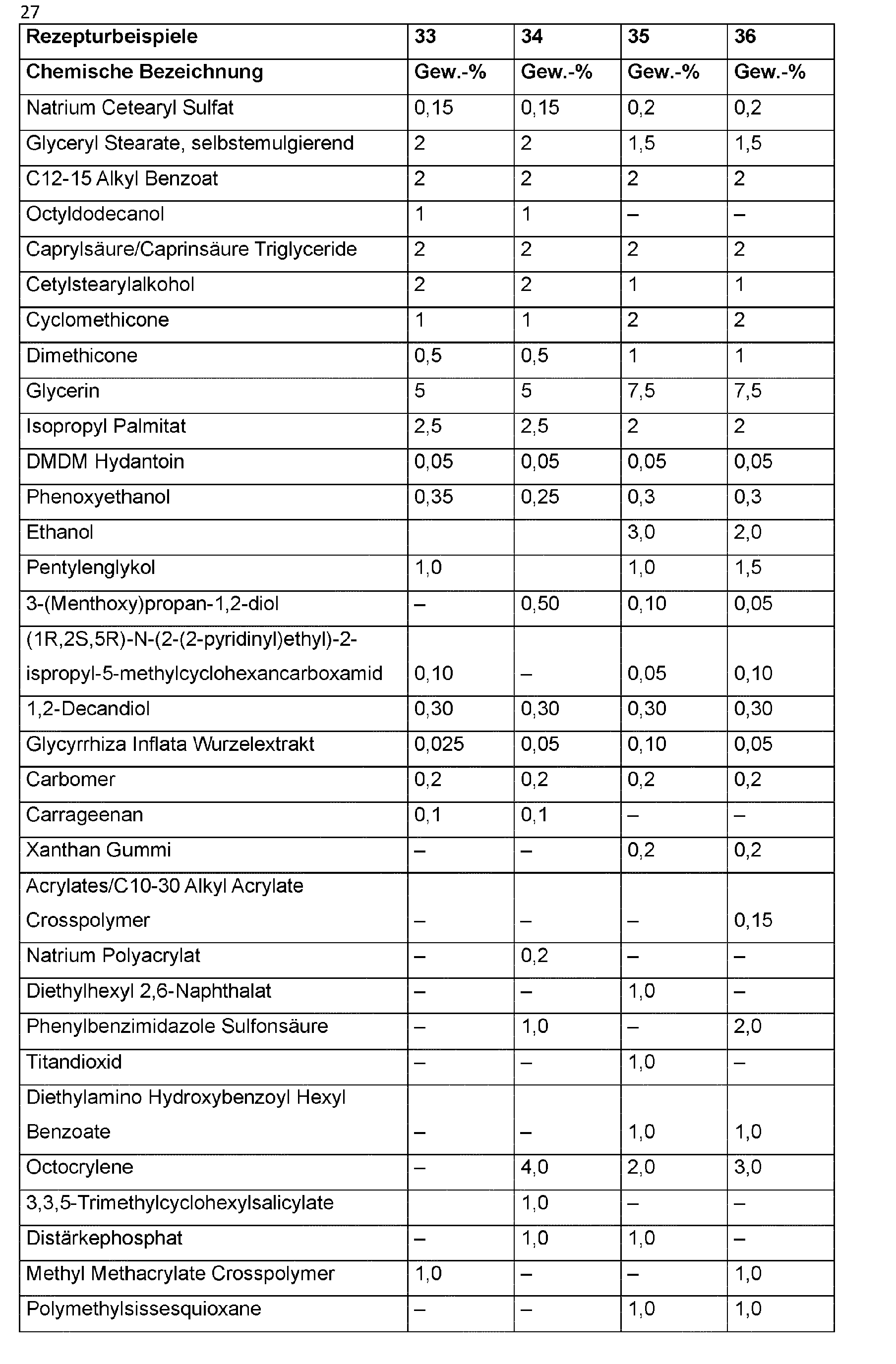

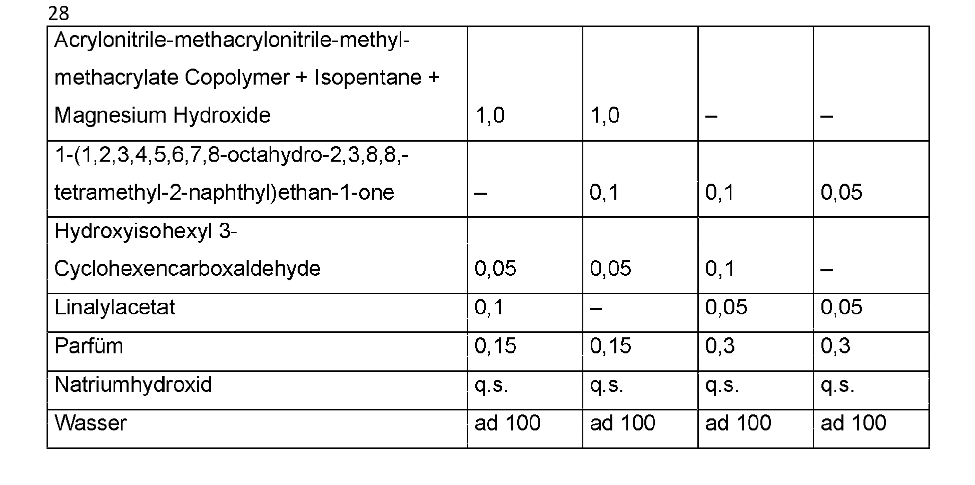

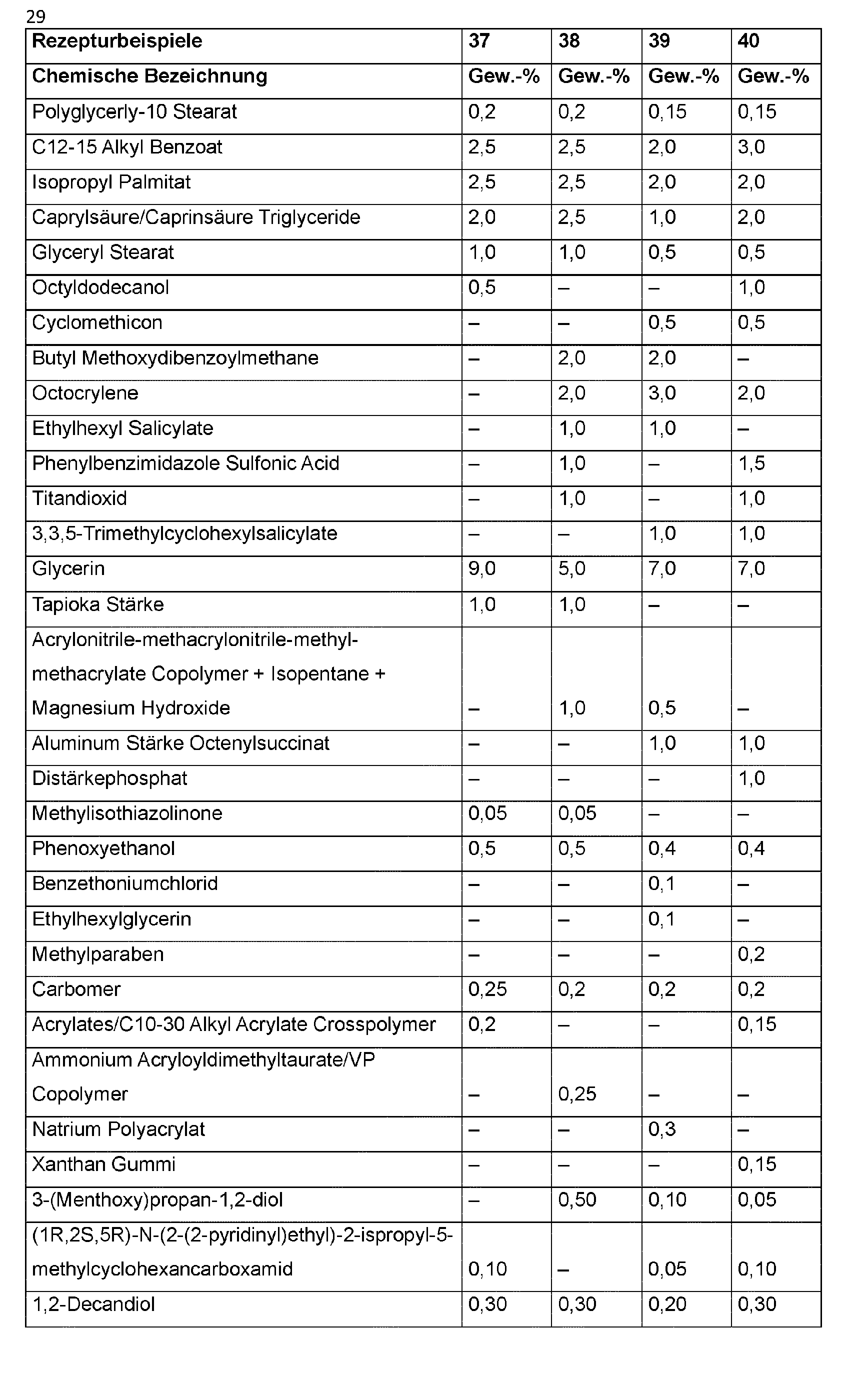

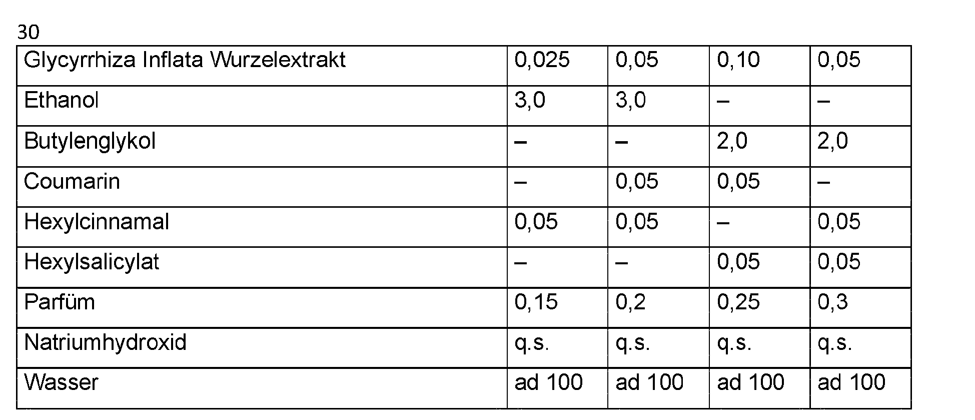

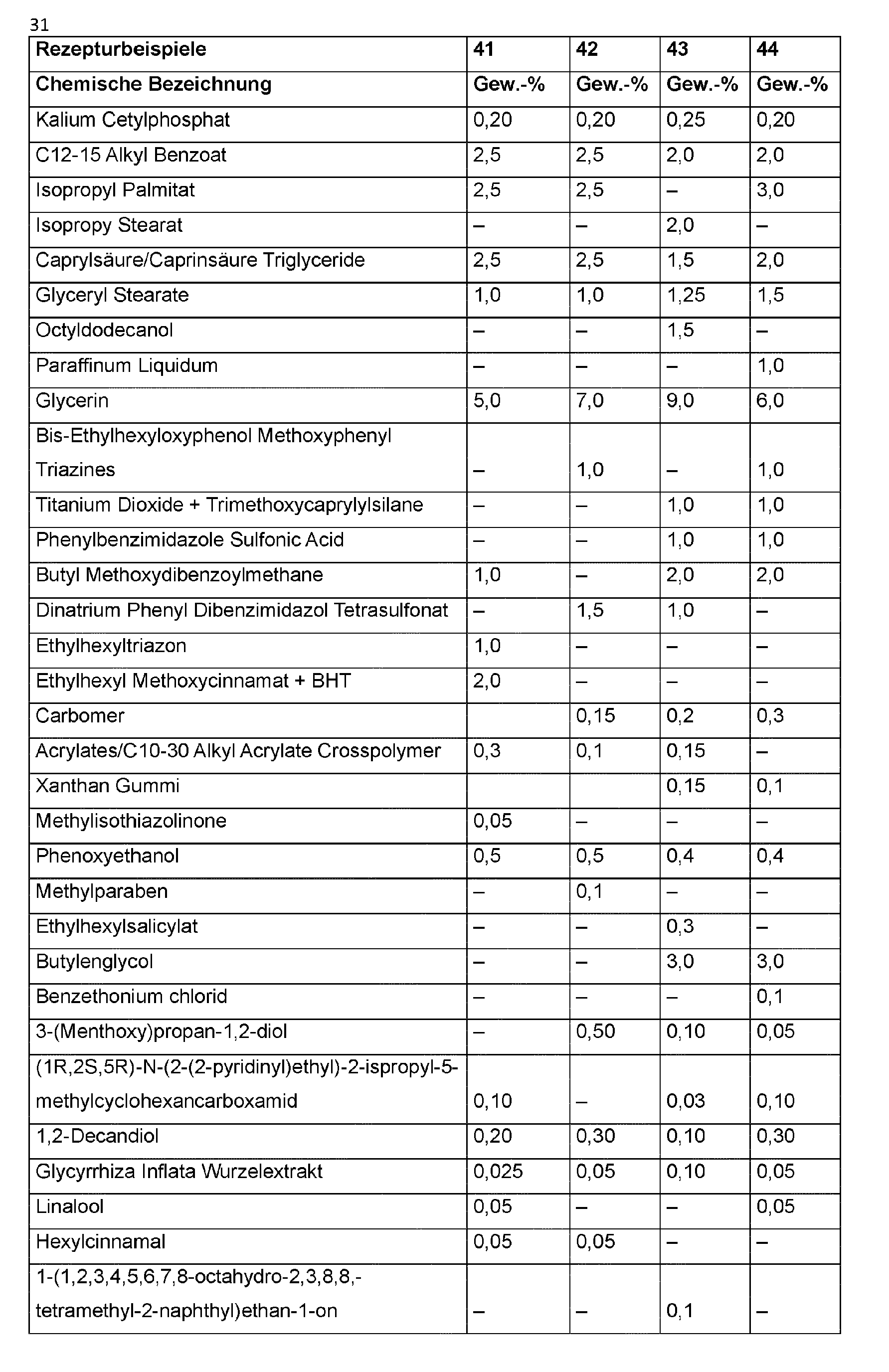

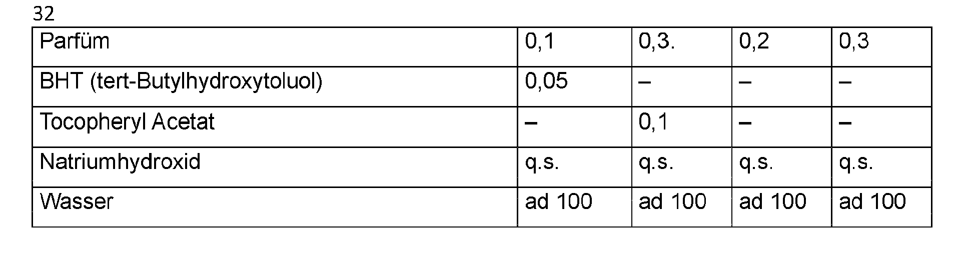

## Patentansprüche

1. Kosmetische Zubereitungen enthaltend Wirkstoffkombinationen aus Licochalcon A, Coolant XL ((1R,2S,5R)-N-(2-(2-pyridinyl)ethyl)-2-ispropyl-5-methylcyclohexancarboxamid) und Cooling Agent 10 (Menthoxypropandiol) und 1,2-Decandiol.

2. Zubereitungen nach Anspruch 1, **dadurch gekennzeichnet, dass** sie eine oder mehrere antierythematös wirksame bzw. entzündungshemmende Substanzen enthalten.

3. Zubereitungen nach einem der vorstehenden Ansprüche, **dadurch gekennzeichnet, dass** als antierythematös oder anti-entzündliche wirksame Substanzen Lignan, Chroman- oder Isoflavonoide, pentacyclische Triterpene (z. B. Ursolsäure, Glycyrrhetinsäure), Hamamelis Kamillenextrakte oder Bisabolol, Allantoin, Calendula Extrakte und/oder Panthenol gewählt werden.

## Claims

1. Cosmetic preparations comprising active ingredient combinations of licochalcone A, coolant XL ((1R,2S,5R)-N-(2-(2-pyridinyl)ethyl)-2-isopropyl-5-methylcyclohesxanecar-boxamide) and cooling agent 10 (menthoxypropanediol) and 1,2-decanediol.

2. Preparations according to Claim 1, **characterized in that** said preparations comprise one or more anti-erythematous or anti-inflammatory substances.

3. Preparations according to either of the preceding claims, **characterized in that** the anti-erythematous or anti-inflammatory substances selected are lignan, chromane or isoflavonoids, pentacyclic triterpenes (e.g. ursolic acid, glycyrrhetic acid), hamamelis chamomile extracts or bisabolol, allantoin, calendula extracts and/or panthenol.

## Revendications

1. Préparations cosmétiques contenant des associations de substances actives à base de licochalcone A, d'agent rafraîchissant XL ((1 R,2S,5R)-N-(2-(2-pyridinyl)éthyl)-2-isopropyl-5-méthylcyclohexanecarboxamide) et d'agent rafraîchissant 10 (menthoxypropanediol) et de 1,2-décanediol.

2. Préparations selon la revendication 1, **caractérisées en ce qu'**elles contiennent une ou plusieurs substances anti-inflammatoires ou à activité anti-érythémateuse.

3. Préparations selon l'une quelconque des revendications précédentes, **caractérisées en ce qu'**en tant que substances à activité anti-inflammatoire ou anti-érythémateuse sont choisis un lignane, des chromane- ou isoflavonoïdes, des triterpènes pentacycliques (par exemple l'acide ursolique, l'acide glycyrrhétinique), des extraits de camomille hamamélis ou le bisabolol, l'allantoïne, des extraits de calendule et/ou le panthénol.
